(19) **Europäisches Patentamt / European Patent Office / Office européen des brevets**

(11) **EP 3 915 473 B1**

(12) **EUROPEAN PATENT SPECIFICATION**

(45) Date of publication and mention
of the grant of the patent:
**05.07.2023 Bulletin 2023/27**

(21) Application number: **19911160.0**

(22) Date of filing: **24.01.2019**

(51) International Patent Classification (IPC):
**A61B 5/11** (2006.01)

(52) Cooperative Patent Classification (CPC):
**A61B 5/112; A61B 5/1121;** A61B 5/6829;
A61B 2505/09

(86) International application number:
**PCT/JP2019/002214**

(87) International publication number:
**WO 2020/152818 (30.07.2020 Gazette 2020/31)**

(54) **INFORMATION PROCESSING PROGRAM, INFORMATION PROCESSING METHOD, AND INFORMATION PROCESSING SYSTEM**

INFORMATIONSVERARBEITUNGSPROGRAMM, INFORMATIONSVERARBEITUNGSVERFAHREN UND INFORMATIONSVERARBEITUNGSSYSTEM

PROGRAMME DE TRAITEMENT D'INFORMATIONS, PROCÉDÉ DE TRAITEMENT D'INFORMATIONS ET SYSTÈME DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.12.2021 Bulletin 2021/48**

(73) Proprietor: **FUJITSU LIMITED**
**Kanagawa 211-8588 (JP)**

(72) Inventors:
• **OYA, Takuro**
**Kawasaki-shi, Kanagawa 211-8588 (JP)**
• **MAEDA, Kazuho**
**Kawasaki-shi, Kanagawa 211-8588 (JP)**
• **HOTTA, Shinji**
**Kawasaki-shi, Kanagawa 211-8588 (JP)**
• **AKI, Michihiko**
**Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 257 437        WO-A1-2017/216103**

JP-A- 2009 545 416      JP-A- 2017 213 175
US-A1- 2018 263 532

• **RAMPP ALEXANDER ET AL: "Inertial Sensor-Based Stride Parameter Calculation From Gait Sequences in Geriatric Patients", IEEE TRANSACTIONS ON BIOMEDICAL ENGINEERING, IEEE, USA, vol. 62, no. 4, 1 April 2015 (2015-04-01), pages 1089-1097, XP011575709, ISSN: 0018-9294, DOI: 10.1109/TBME.2014.2368211 [retrieved on 2015-03-17]**
• **PENDHARKAR GITA ET AL: "Automated method to distinguish toe walking strides from normal strides in the gait of idiopathic toe walking children from heel accelerometry data", GAIT & POSTURE, vol. 35, no. 3, 1 March 2012 (2012-03-01), pages 478-482, XP028903876, ISSN: 0966-6362, DOI: 10.1016/J.GAITPOST.2011.11.011**
• **PENDHARKAR, GITA et al.: "Automated method to distinguish toe walking strides from normal strides in the gait of idiopathic toe walking children from heel accelerometry data", Gait & Posture, vol. 35, no. 3, March 2012 (2012-03), pages 478-482, XP028903876, DOI: 10.1016/j.gaitpost.2011.11.011**

**(Cont. next page)**

- **Catie Christensen, Haddad Amanda, Maus Elizabeth: "Reliability and Validity of the 50-ft Walk Test for Idiopathic Toe Walking", Pediatric physical therapy, vol. 29, no. 3, July 2017 (2017-07), pages 238-243, XP055728435,**
- **Catie Christensen, Haddad Amanda, Maus Elizabeth: "Validity of an Accelerometer Used to Measure Step Count in Children With Idiopathic Toe Walking", Pediatric physical therapy, vol. 29, no. 2, April 2017 (2017-04), pages 153-157, XP055728437,**

## Description

[Field]

The present invention relates to an information processing program, an information processing method, and an information processing system.

[Background Art]

In a patient with stroke, peripheral nerve palsy, a Parkinson's disease, or the like, to compensate for abnormality in a desired motion, another part may perform a movement (compensatory movement) to achieve the desired motion instead.

For example, a patient with peripheral nerve palsy may have a gait disturbance called toe gait (equinus gait or tiptoe gait). The toe gait is a compensatory movement for walking with only a toe without putting a heel on the ground because an Achilles' tendon is rigid.

Conventionally, a system that evaluates a degree of equinus is known (refer to, for example, PTL 1).

PTL 2 relates to a method for analyzing human gait comprising: providing data representing the 3D-movement of a foot of said subject over time; identifying within said data first data segments that each represent of at least one stride; determining one or more stride features for each of said first data segments; and defining one or more clusters on the basis of at least one stride feature of said one or more stride features.

PTL 3 relates to a method for computing clinically relevant temporal and spatial gait parameters, wherein an accelerometer and a gyroscope are positioned laterally below' each ankle joint and temporal gait events were detected by searching for characteristic features in the signals; further to calculate stride length, the gravity compensated accelerometer signal is double integrated, and sensor drift is modeled using a piece-wise defined linear function.

PTL 4 relates to an automated method for assessing the gait in Idiopathic Toe Walking children using a dual axis accelerometer, wherein heel acceleration data is recorded from the gait of the children using boots embedded with the sensor in the heel and is interfaced to a handheld oscilloscope.

PTL 5 relates to the detection of toe walking include a gait monitoring device that includes one or more sensors usable to collect gait data relative to the gait monitoring device and vibration circuitry that includes a vibrating motor usable to provide haptic feedback in the form of a vibration.

[Citation List]

[Patent Literature]

[PTL 1] International Publication Pamphlet No. WO 2013/108306 [PTL 2] EP 3 257 437 A1

[PTL 3] RAMPP ALEXANDER ET AL: "Inertial Sensor-Based Stride Parameter Calculation From Gait Sequences in Geriatric Patients", IEEE TRANSACTIONS ON BIOMEDICAL ENGINEERING, IEEE, USA, vol. 62, no. 4, 1 April 2015

[PTL 4] PENDHARKAR GITA ET AL: "Automated method to distinguish toe walking strides from normal strides in the gait of idiopathic toe walking children from heel accelerometry data", GAIT & POSTURE, vol. 35, no. 3, pages 478-482, [PTL5] US 2018/263532 A1

[Summary of Invention]

[Technical Problem]

However, with conventional technology, the degree of equinus of a standing patient is evaluated, and thus it is not possible to analyze a toe gait.

In one aspect, an object of the present invention is to provide an information processing program, an information processing method, and an information processing system that are capable of calculating an index for accurately determining a toe gait.

[Solution to Problem]

In one aspect, an information processing program is a program that causes a computer to execute processing of: acquiring information regarding an angular velocity around a first axis that extends in a right and left direction of a person and an acceleration in a front and back direction of the person during walking that are detected by a sensor attached to an ankle of the person; detecting a timing when a foot that is off a ground touches the ground based on change in the angular velocity over time around the first axis; specifying, as a time of interest, a predetermined time before and after the detected timing; and calculating an index and using the index to determine whether or not the person is performing a toe gait based on change in the acceleration in the front and back direction within the time of interest.

[Advantageous Effects of Invention]

It is possible to calculate an index for accurately determining a toe gait.

[Brief Description of Drawings]

[FIG. 1] FIG. 1 is a diagram schematically illustrating a configuration of an information processing system according to an embodiment.
[FIG. 2] FIG. 2A is a diagram illustrating a hardware configuration of a physical therapist terminal and a doctor terminal, and FIG. 2B is a diagram illustrating a hardware configuration of a server.

[FIG. 3] FIG. 3 is a diagram for describing a Rancho Los Amigos method.

[FIG. 4] FIG. 4 is a functional block diagram of the server.

[FIG. 5] FIG. 5 is a diagram illustrating an X-axis and a Y-axis set in a sensing device.

[FIG. 6] FIG. 6A is a diagram illustrating data of change in an angular velocity over time around the X-axis, and FIG. 6B is a diagram illustrating data of change in an acceleration over time in a Y-axis direction.

[FIG. 7] FIGs. 7A to 7C are diagrams for describing data and a movement of a leg during a normal gait.

[FIG. 8] FIGs. 8A to 8C are diagrams for describing data and a movement of a leg during a toe gait.

[FIG. 9] FIG. 9 is a flowchart illustrating preprocessing by the sensing device.

[FIG. 10] FIG. 10 is a flowchart illustrating data analysis processing by the server.

[FIG. 11] FIGs. 11A and 11B are diagrams for describing the processing in FIG. 10.

[FIG. 12] FIG. 12A is a graph illustrating a feature amount of a subject A, and FIG. 12B is a graph illustrating a feature amount of a subject B.

[FIG. 13] FIGs. 13A to 13B are diagrams for describing a modification.

[Description of Embodiments]

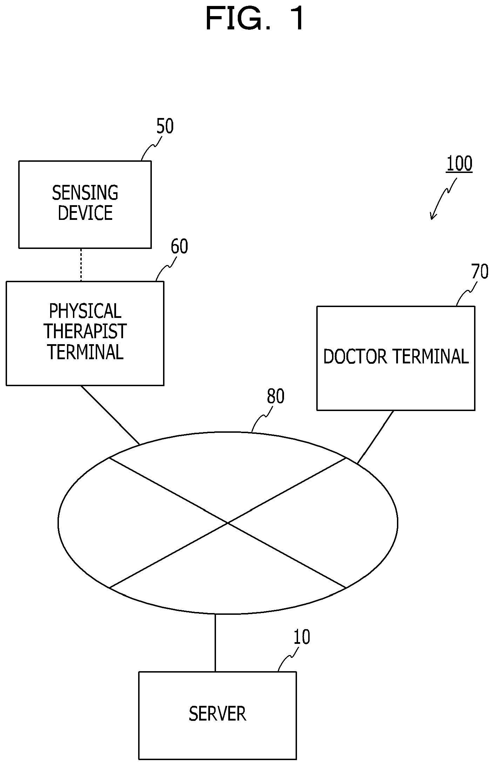

Hereinafter, an embodiment of an information processing system will be described in detail based on FIGs. 1 to 12B. FIG. 1 schematically illustrates a configuration of an information processing system 100 according to an embodiment.

As illustrated in FIG. 1, the information processing system 100 includes a sensing device 50 as a sensor, a physical therapist terminal 60, a doctor terminal 70, and a server 10. The server 10, the physical therapist terminal 60, and the doctor terminal 70 are connected to a network 80 such as the Internet.

The sensing device 50 includes an angular velocity sensor, an acceleration sensor, a control unit that controls detection of both sensors, and a memory that stores detection results of both sensors. The sensing device 50 is provided on both ankles of a patient when the patient performs a walk test, which is one of motor function tests, under instructions of a physical therapist, and detects change in an angular velocity and acceleration over time. It is assumed that the sensing device 50 is also provided with input buttons for inputting start and end of measurement.

The physical therapist terminal 60 is a terminal used by a physical therapist, such as a personal computer (PC) or a tablet-type terminal. The physical therapist terminal 60 is connected to the sensing device 50 to acquire detection results (data indicating change in an angular velocity over time and data indicating change in an acceleration over time) of the sensing device 50. Furthermore, the physical therapist terminal 60 transmits the acquired data to the server 10 via the network 80.

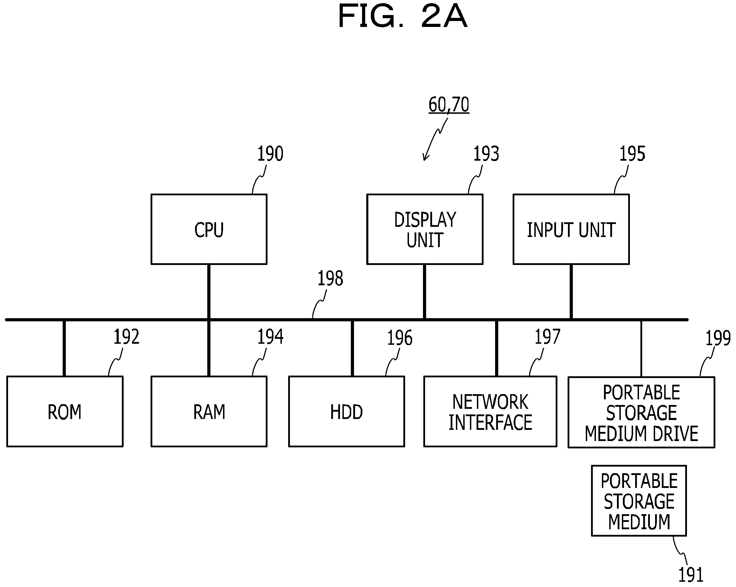

Here, FIG. 2A illustrates a hardware configuration of the physical therapist terminal 60. As illustrated in FIG. 2A, the physical therapist terminal 60 includes a central processing unit (CPU) 190, a read only memory (ROM) 192, a random access memory (RAM) 194, a storage unit (here, hard disk drive (HDD)) 196, a network interface 197, a display unit 193, an input unit 195, and a portable storage medium drive 199 capable of reading data stored in a portable storage medium 191. The display unit 193 includes a liquid crystal display, and the input unit 195 includes a keyboard, a mouse, and a touch panel. Each of these components of the physical therapist terminal 60 is connected to a bus 198. Furthermore, the physical therapist terminal 60 includes a communication unit that communicates wirelessly or by wire with the sensing device 50.

Returning to FIG. 1, the doctor terminal 70 is a terminal used by a doctor, such as a PC. The doctor terminal 70 is a terminal that displays information transmitted from the server 10 and presents the information to a doctor. As illustrated in FIG. 2A, the doctor terminal 70 has a similar hardware configuration to that of the physical therapist terminal 60. A doctor refers to information displayed on the doctor terminal 70 to confirm a state of a toe gait of a patient, examine a treatment policy, and confirm an effect of medication. Here, the toe gait is a compensatory movement performed by a patient with peripheral nerve palsy or the like during walking, and means walking with only a toe without putting a heel on the ground.

The server 10 calculates a feature amount related to a toe gait of a patient based on data indicating change in an angular velocity over time and data indicating change in an acceleration over time that are acquired from the physical therapist terminal 60. Furthermore, the server 10 outputs the calculated feature amount to the doctor terminal 70.

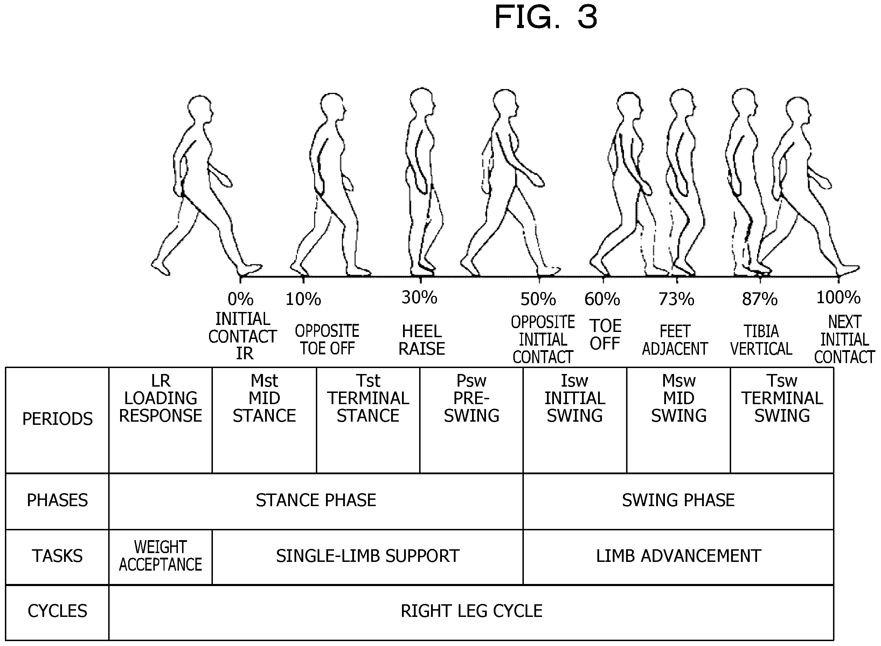

Here, when calculating a feature amount related to a toe gait, the server 10 of the present embodiment calculates an index in accordance with a walking cycle used in a current clinical site. The most used walking cycle in a clinical site is a walking cycle called "Rancho Los Amigos method" as illustrated in FIG. 3, which was developed by Kirsten Götz-Neumann, a German physical therapist at the Rancho Los Amigos National Rehabilitation Center. In the Rancho Los Amigos method, a walking cycle is divided into eight phases. The walking cycle means from when a leg touches the ground until the same leg touches the ground again. Furthermore, the eight phases of the walking cycle are initial contact (IR), loading response (LR), mid stance (Mst), terminal stance (Tst), pre-swing (Psw), initial swing (Isw), mid swing (Msw), and terminal swing (Tsw). Among these, a time from IR to Psw is called a stance phase, and a time from Isw to Tsw is called a swing phase. In the stance phase, a target leg (right leg in FIG. 3) is on a floor (ground), and in the swing phase, the target leg is off the floor (ground). In the present embodiment, an initial contact timing of the walking cycle is focused on to calculate a feature amount related to a toe gait.

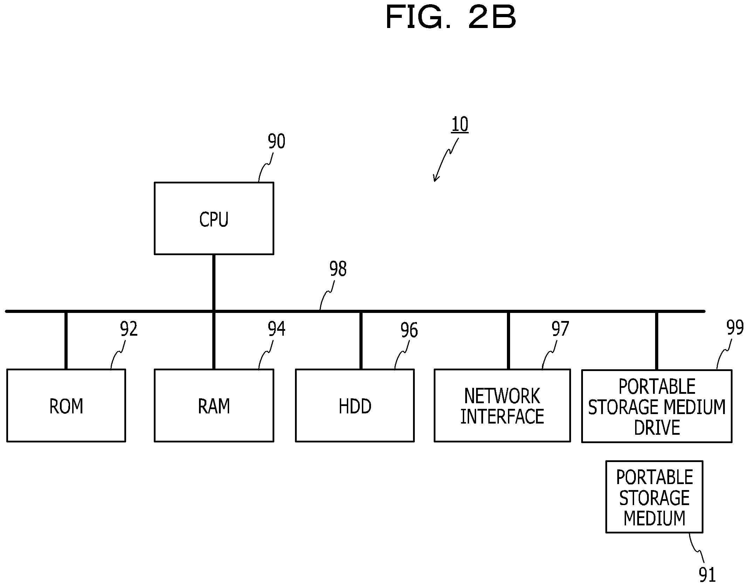

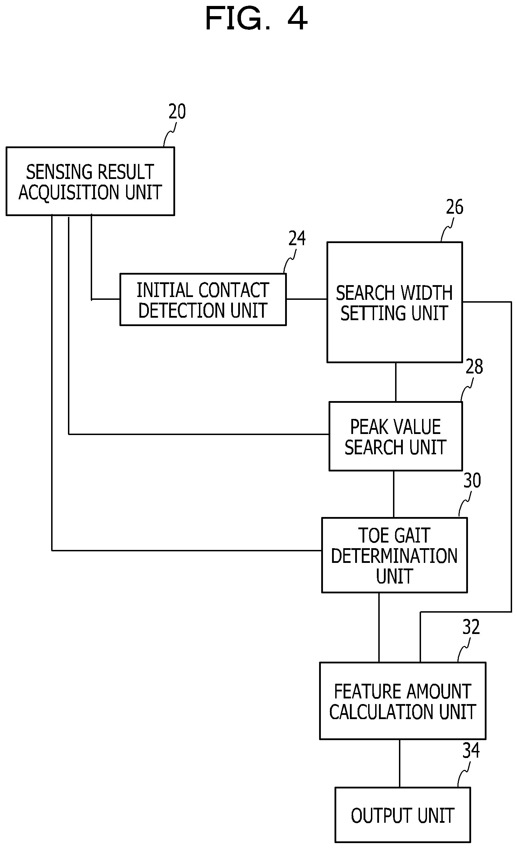

FIG.2B illustrates a hardware configuration of the server 10. The server 10 includes a CPU 90, a ROM 92, a RAM 94, a storage unit (here, HDD) 96, a network interface 97, and a portable storage medium drive 99. Each of these components of the server 10 is connected to a bus 98. In the server 10, the CPU 90 executes a program (including an information processing program) stored in the ROM 92 or the HDD 96 or a program (including an information processing program) read by the portable storage medium drive 99 from a portable storage medium 91, to implement a function of each unit illustrated in FIG. 4. Note that the function of each unit in FIG. 4 may be implemented by, for example, an integrated circuit such as an application specific integrated circuit (ASIC) or a field programmable gate array (FPGA).

FIG. 4 illustrates a functional block diagram of the server 10. In the server 10, the CPU 90 executes a program to implement functions as a sensing result acquisition unit 20 as an acquisition unit, an initial contact detection unit 24 as a detection unit, a search width setting unit 26 as a specification unit, a peak value search unit 28, a toe gait determination unit 30, a feature amount calculation unit 32, and an output unit 34.

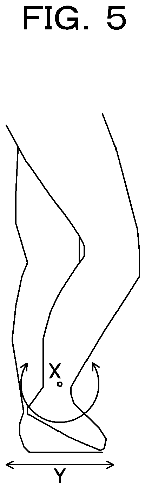

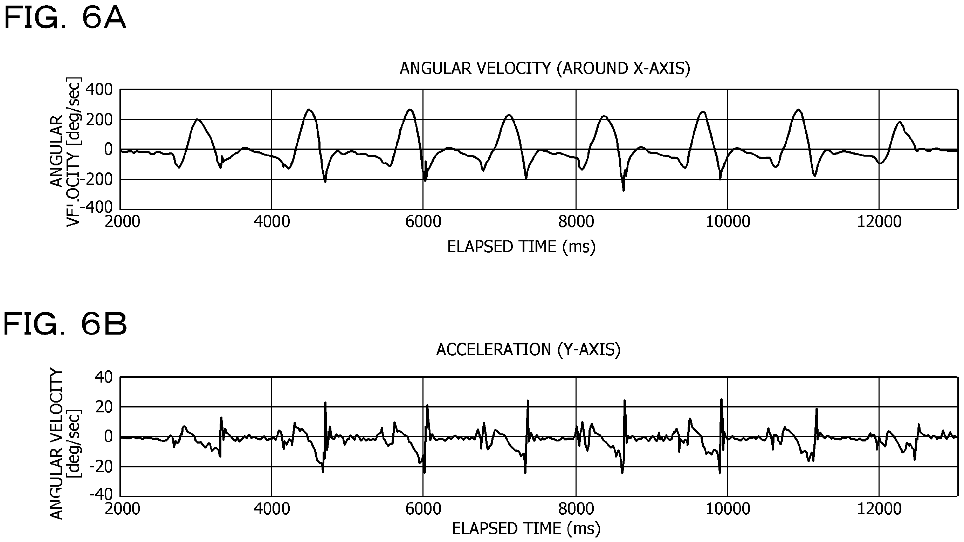

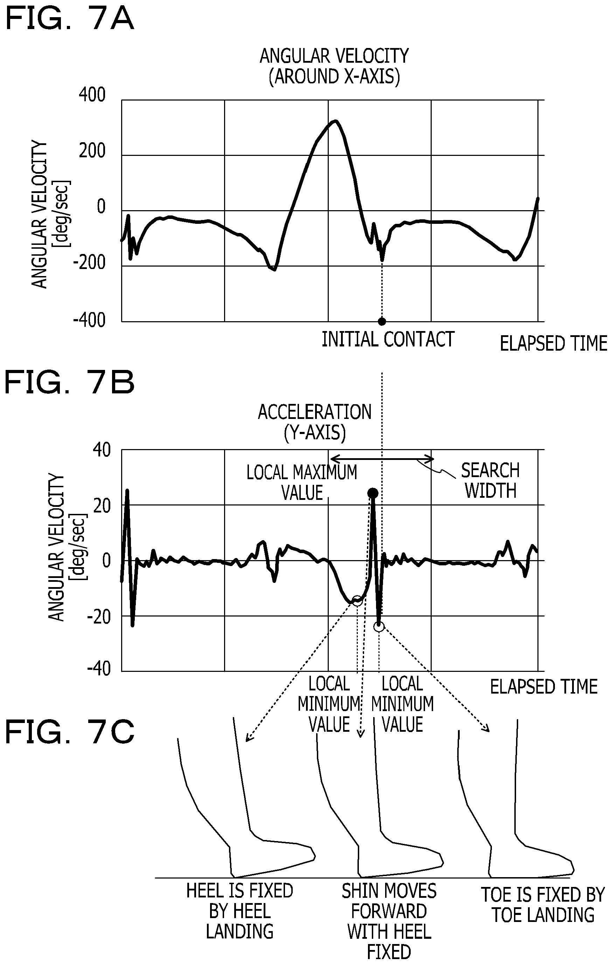

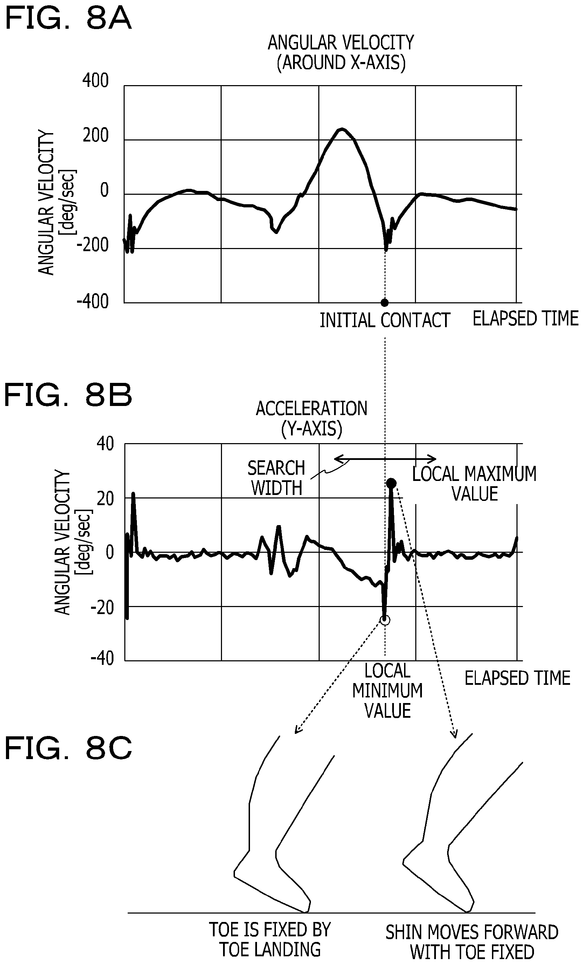

The sensing result acquisition unit 20 acquires, from the physical therapist terminal 60, data of change in an angular velocity over time and data of change in an acceleration over time that are detected by the sensing device 50. Here, the sensing device 50 detects an angular velocity around a first axis (X-axis) (angular velocity in a traveling direction) extending in a right and left direction of a patient, and an acceleration in a second axis (Y-axis) direction (acceleration in the traveling direction) in a front and back direction of the patient, which are indicated by arrows in FIG. 5. Note that data of change in an angular velocity over time around the X-axis acquired in one walk test is, for example, data as illustrated in FIG. 6A, and data of change in an acceleration over time in the Y-axis direction acquired in one walk test is, for example, data as illustrated in FIG. 6B. Note that FIGs. 7A and 7B are enlarged diagrams illustrating part of data of change in an angular velocity over time around the X-axis during a normal gait and part of data of change in an acceleration over time in the Y-axis direction during a normal gait. Furthermore, FIGs. 8A and 8B are enlarged diagrams illustrating part of data of change in an angular velocity over time around the X-axis during a toe gait and part of data of change in an acceleration over time in the Y-axis direction during a toe gait.

The initial contact detection unit 24 detects a timing when a foot that is off a floor (ground) touches the floor (ground) (initial contact) based on data of change in an angular velocity over time around the X-axis. As illustrated in FIGs. 7A and 8A, as the initial contact timing, the swing phase specification unit 22 detects a timing when the value of the angular velocity around the X-axis indicates a local minimum value after the value increases significantly. Note that the swing phase specification unit 22 detects the initial contact timing by, for example, differentiating the data of change in the angular velocity over time around the X-axis.

The search width setting unit 26 sets, as a search width, a predetermined time before and after initial contact. As the search width, the search width setting unit 26 sets, for example, a total time of 500 ms obtained by adding 250 ms immediately before and 250 ms immediately after an initial contact timing. Note that the search width setting unit 26 may set the search width based on a local maximum value of an angular velocity around the X-axis (local maximum value immediately before the initial contact) instead of the initial contact timing. Note that, in FIGs. 7B and 8B, the search width is indicated by double-headed arrows.

The peak value search unit 28 searches for peak values of an acceleration in the Y-axis direction within a range of a search width set by the search width setting unit 26. Note that, in the present embodiment, the "peak values" are assumed to be a local maximum value of an acceleration value that exceeds a predetermined range (for example, -10 to 10 m/s$^2$) and a local minimum value of the acceleration value that is lower than the predetermined range. Furthermore, the peak value search unit 28 searches for a local maximum value that exceeds a predetermined range, and then searches whether or not there is a local minimum value that is lower than the predetermined range before and after the local maximum value. In the example of FIG. 7B, a local minimum value, a local maximum value, and a local minimum value are searched for as peak values within the search width. Furthermore, in the example of FIG. 8B, a local minimum value and a local maximum value are searched for as peak values within the search width. Note that the predetermined range is assumed to be adjustable by a doctor or a physical therapist. For example, in a case where it is not possible to accurately detect a toe gait of a patient, a doctor or a physical therapist need only make an adjustment to narrow (or widen) the predetermined range to increase (or decrease) sensitivity of the sensors.

In a case where a "local minimum value", a "local maximum value", and a "local minimum value" are searched for as peak values within a search width as illustrated in FIG. 7B as a result of search by the peak value search unit 28, the toe gait determination unit 30 determines that a "normal gait" is performed. On the other hand, in a case where a "local minimum value" and a "local maximum value" are searched for as peak values within a search width as illustrated in FIG. 8B as a result of search by the peak value search unit 28, the toe gait determination unit 30 determines that a "toe gait" is performed.

FIG. 7C schematically illustrates a movement of a leg during a normal gait. During the normal gait illustrated in FIG. 7C, before and after initial contact, a heel is fixed by heel landing, a shin moves forward with the heel fixed, and a toe is fixed by toe landing. Thus, in a case where a normal gait is performed, local minimum values appear both before and after a local maximum value, as illustrated in FIG. 7B.

On the other hand, FIG. 8C schematically illustrates a movement of a leg during a toe gait. During the toe gait illustrated in FIG. 8C, before and after initial contact, a toe is fixed by toe landing, and then a shin moves forward with the toe fixed. Thus, in a case where a toe gait is performed, although a local minimum value appears before a local maximum value, a local minimum value does not appear after the local maximum value, as illustrated in FIG. 8B.

The feature amount calculation unit 32 calculates a feature amount related to a toe gait based on a determination result of the toe gait determination unit 30. Specifically, the feature amount calculation unit 32 sets the number of search widths (for example, the number of times of initial contact) set by the search width setting unit 26 as the number of steps S and the number of times T determined to be a toe gait, and calculates, from the number of steps S and the number of times T, a feature amount C based on the following Equation (1).

$$C = T/S \ldots (1)$$

Note that, as can be seen from the above Equation (1), the feature amount C is a percentage of the number of steps of a toe gait to the total number of steps.

The output unit 34 outputs a feature amount C calculated by the feature amount calculation unit 32 to the doctor terminal 70.

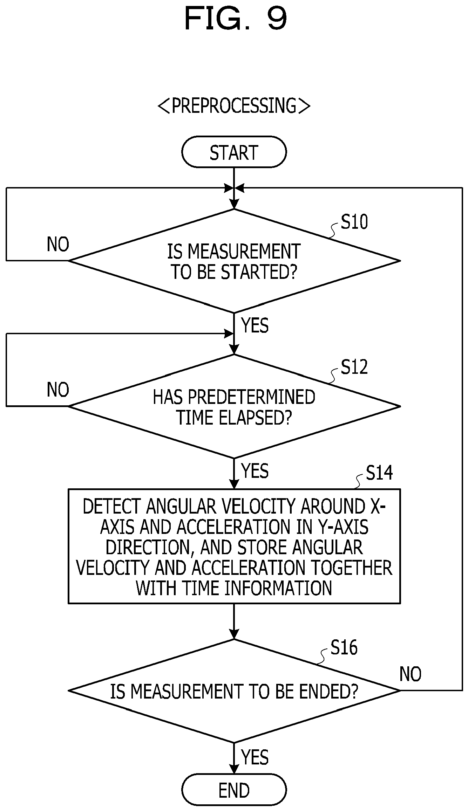

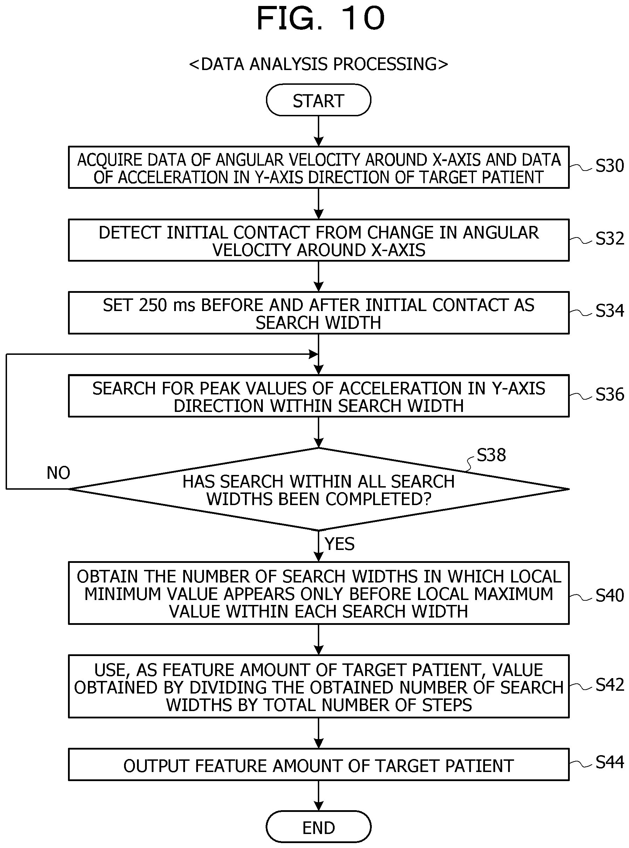

Next, flows of preprocessing by the sensing device 50 and data analysis processing by the server 10 will be described with reference to flowcharts in FIGs. 9 and 10.

(Regarding Preprocessing by Sensing Device 50)

FIG. 9 illustrates a flowchart of the preprocessing by the sensing device 50. In this preprocessing, the sensing device 50 acquires data of change in an angular velocity and acceleration over time while a patient is performing a walk test under instructions of a physical therapist. As a premise of the processing in FIG. 9, it is assumed that the patient has the sensing devices 50 attached to both ankles and is ready for the walk test. The walk test is performed for a distance of 10 m, for example, and the sensing devices 50 detect angular velocities and accelerations of both legs while the patient is walking straight for the distance of 10 m.

In the processing of FIG. 9, first, in Step S10, the control unit of the sensing device 50 stands by until an instruction to start measurement is input. For example, in a case where the input button for inputting measurement start provided in the sensing device 50 is pressed by the physical therapist or the like, the control unit advances the processing to Step S12.

When the processing proceeds to Step S12, the control unit stands by until a predetermined time elapses. The predetermined time here means a measurement interval, and is assumed to be about several ms to several tens of ms.

When the predetermined time has elapsed and the processing proceeds to Step S14, the control unit detects an angular velocity around the X-axis and an acceleration in the Y-axis direction by using the angular velocity sensor, and stores the angular velocity and the acceleration in the memory together with time information.

Next, in Step S16, the control unit determines whether or not to end the measurement. For example, in a case where the input button for inputting measurement end is pressed by the physical therapist or the like, the determination in Step S16 is positive, but in a case where the input button is not pressed, the processing returns to Step S12. Thereafter, the control unit repeatedly executes the processing and determination in Steps S12 to S16 until the determination in Step S16 becomes positive. Then, when the determination in Step S16 is positive, the entire processing in FIG. 9 is ended.

As described above, by performing the processing of FIG. 9, it is possible to store, in the memory, data of change in the angular velocity over time around the X-axis and data of change in the acceleration over time in the Y-axis direction of a leg during the walk test performed by the patient (for example, the data in FIGs. 6A and 6B). Note that the sensing devices 50 are provided on both ankles during the walk test. Thus, after the walk test is completed, the data of FIGs. 6A and 6B will be acquired for both legs.

Note that, the data stored in the memory by the processing of FIG. 9 is transmitted to the physical therapist terminal 60 when the sensing device 50 is connected to the physical therapist terminal 60. It is assumed that the physical therapist transmits the data to the server 10 after associating each piece of data with the patient on the physical therapist terminal 60.

(Regarding Data Analysis Processing by Server 10)

Next, the data analysis processing by the server 10 will be described in detail based on FIG. 10. The processing of FIG. 10 is assumed to be started at a timing when data of change in an angular velocity and acceleration over time of a patient (target patient) is transmitted from the physical therapist terminal 60, as an example.

In the processing of FIG. 10, first, in Step S30, the sensing result acquisition unit 20 acquires data of change in an angular velocity over time around the X-axis and data of change in an acceleration over time in the Y-axis direction of the target patient. Here, it is assumed that the sensing result acquisition unit 20 has acquired the data in FIGs. 6A and 6B as data of one leg of the target patient.

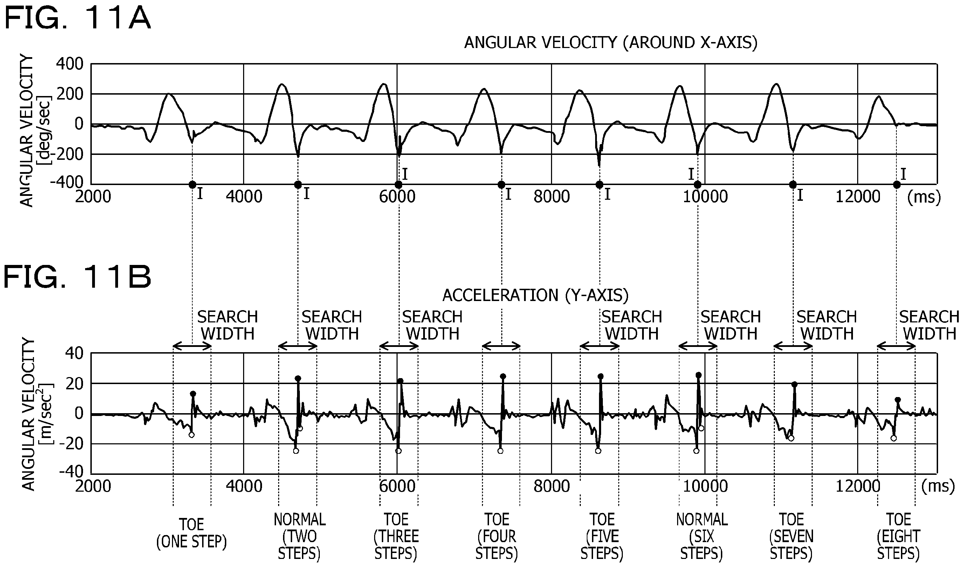

Next, in Step S32, the initial contact detection unit 24 detects initial contact from the change in the angular velocity around the X-axis. From the data in FIG. 6(a), the initial contact is detected a plurality of times as indicated by a reference sign I in FIG. 11A.

Next, in Step S34, the search width setting unit 26 sets 250 ms before and after the initial contact as a search width. From the data in FIG. 6A, a plurality of search widths is set as indicated by double-headed arrows in FIG. 11B.

Next, in Step S36, the peak value search unit 28 specifies one search width and searches for peak values of the acceleration in the Y-axis direction within the specified search width. Next, in Step S38, the peak value search unit 28 determines whether or not search within all the search widths has been completed. In a case where the determination in Step S38 is negative, the processing in Step S36 is repeatedly executed until the determination in Step S38 becomes positive. Then, when the determination in Step S38 is positive, the processing proceeds to Step S40. Note that, it is assumed that, at the stage of proceeding to Step S40, local maximum values indicated in FIG. 11B by black circles (●) are searched for, and local minimum values indicated by white circles (○) are searched for before and after the local maximum values.

When the processing proceeds to Step S40, the toe gait determination unit 30 obtains the number of search widths in which a local minimum value appears only before a local maximum value within each search width, for example, the number of toe gaits. In the example of FIG. 11B, the number of toe gaits is 6 steps.

Next, in Step S42, the feature amount calculation unit 32 uses, as a feature amount of the target patient, a value obtained by dividing the obtained number of search widths (the number of steps of a toe gait) by the total number of steps (the total number of search widths) (refer to the above Equation (1)). For example, in the example of FIG. 11B, the feature amount C = 6/8 = 0.75.

Next, in Step S44, the output unit 34 outputs the feature amount of the target patient to the doctor terminal 70. Thus, the entire processing in FIG. 10 ends. Note that the feature amount calculation unit 32 may separately obtain feature amounts from data of each of the left leg and the right leg, or may obtain one feature amount from data of both legs.

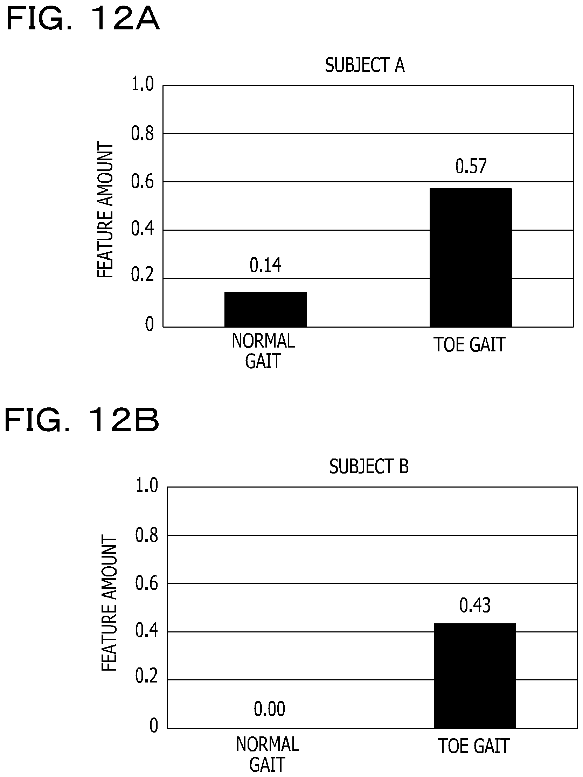

FIGs. 12A and 12B are graphs illustrating feature amounts output when subjects A and B performed a walk test, with two healthy persons as the subjects A and B. In this walk test, the two subjects A and B were asked to perform a normal gait and a toe gait. Then, feature amounts were calculated based on detection results of the sensing devices 50 during the walk test.

As illustrated in FIGs. 12A and 12B, although there are individual differences in the magnitude of the value of the feature amount, it can be seen that, for the same subject, the value of the toe gait is larger than that of the normal gait.

Thus, a doctor may confirm whether a toe gait of a patient is improved by observing change in a feature amount for each patient. This enables the doctor to appropriately perform follow-up in a case where a patient is undergoing rehabilitation and confirm an effect of medicine. Furthermore, since the doctor may appropriately perform follow-up and confirm an effect of medicine, the doctor may appropriately construct rehabilitation plans and medication plans, perform clinical trials, and the like. Furthermore, in a case where change in a feature amount of a target patient is similar to that of another patient, the doctor may perform construction of rehabilitation plans and medication plans, and the like with reference to data of treatment executed for the another patient.

As can be seen from the description so far, in the present embodiment, the peak value search unit 28, the toe gait determination unit 30, and the feature amount calculation unit 32 implement a function as a calculation unit that calculates an index (feature amount) used to determine whether or not a patient is performing a toe gait based on change in an acceleration in the Y-axis direction within a search width.

As described in detail above, according to the present embodiment, the sensing result acquisition unit 20 acquires data of change in an angular velocity and acceleration over time of an ankle during walking detected by the sensing device 50 attached to the ankle of a patient. Furthermore, the initial contact detection unit 24 detects an initial contact timing based on change in an angular velocity over time around the X-axis, and the search width setting unit 26 sets a time before and after the initial contact timing as a search width. Then, the toe gait determination unit 30 obtains the number of steps of a toe gait based on an appearance pattern of peak values of an acceleration in the Y-axis direction searched for by the peak value search unit 28 within the search width, and the feature amount calculation unit 32 calculates a feature amount based on the number of steps of a toe gait. Therefore, in the present embodiment, since a toe gait is determined and the feature amount is calculated in accordance with a walking cycle used in a clinical site (Rancho Los Amigos method), a doctor may accurately determine (analyze) whether or not the patient is performing a toe gait based on the feature amount. Furthermore, in the present embodiment, since the sensing device 50 is attached to the ankle of the patient during a walk test, muscles and thick blood vessels are not pressed, unlike in a case where the sensing device 50 is attached to a thigh. Therefore, a burden on the patient may be reduced.

Furthermore, in the present embodiment, a change pattern in a case where a toe gait is performed is a pattern in which, before a local maximum value that exceeds a predetermined reference range, a local minimum value that is lower than the reference range appears, and after the local maximum value, a local minimum value that is lower than the reference range does not appear. Therefore, the toe gait determination unit 30 may accurately determine presence or absence of a toe gait based on a pattern that is likely to appear before and after initial contact in a case where a toe gait is performed.

Note that, in the embodiment described above, the case has been described where the feature amount is calculated based on the change pattern of the acceleration in the Y-axis direction within the search width. However, the present invention is not limited to this. In the embodiment described above, the change pattern of the acceleration in the Y-axis direction within the search width is one of the following patterns (1) and (2).

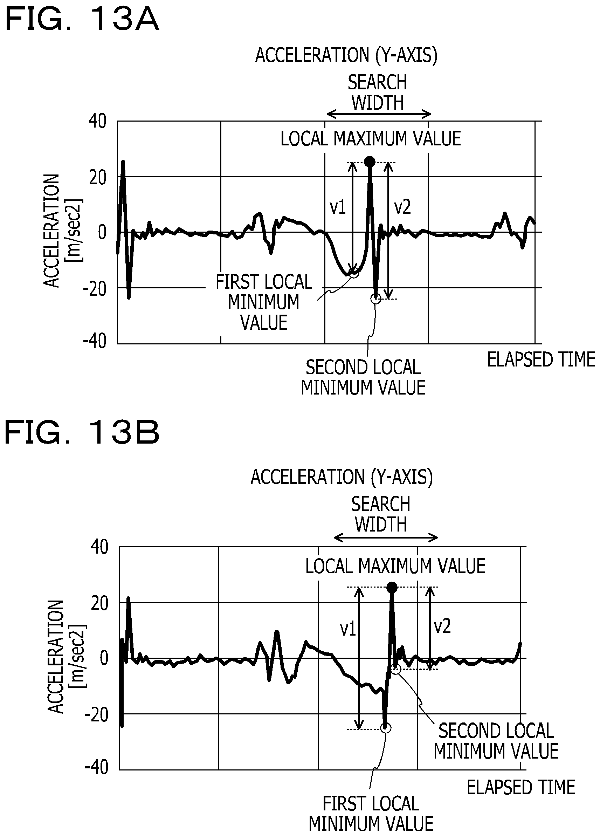

(1) A pattern in which, before a local maximum value that exceeds a predetermined reference range (for example, -10 to 10 $m/s^2$), a first local minimum value that is lower than the reference range appears, and after the local maximum value, a second local minimum value that is lower than the reference range appears (refer to FIG. 13A).
(2) A pattern in which, before a local maximum value that exceeds a predetermined reference range (for example, -10 to 10 $m/s^2$), a first local minimum value that is lower than the reference range appears, and after the local maximum value, a second local minimum value within the reference range appears (refer to FIG. 13B).

Thus, the feature amount calculation unit 32 may calculate, as a feature amount C, a ratio (v1/v2) of a difference v1 between the local maximum value and the first local minimum value and a difference v2 between the local maximum value and the second local minimum value. FIG. 13A is a graph of an acceleration in the Y-axis direction during a normal gait, and FIG. 13B is a graph of an acceleration in the Y-axis direction during a toe gait. A value of the ratio (v1/v2) in the case of the normal gait in FIG. 13A is smaller than a value of the ratio (v1/v2) in the case of the toe gait in FIG. 13B. Thus, also by calculating the ratio (v1/v2) as a feature amount, it is possible to provide a doctor with a feature amount that enables accurate analysis of a toe gait, as in the embodiment described above. Furthermore, the ratio (v1/v2) is a value calculated in accordance with a walking cycle used in a clinical site (Rancho Los Amigos method). From this point as well, presence or absence of a toe gait may be appropriately determined.

Note that the output unit 34 may use an average value of a plurality of ratios (feature amounts) obtained in one walk test, or the like as a feature amount to be output to the doctor terminal 70. Note that, in a case where a second local minimum value is within a reference range (for example, -10 to 10 $m/s^2$) as in FIG. 13B, a value at the origin (0 $m/s^2$) may be used instead of the second local minimum value to calculate the ratio (v1/v2).

Note that, in the embodiment described above, the case has been described where the processing in FIG. 10 is executed by the server 10. However, the present invention is not limited to this. For example, the physical therapist terminal 60 or the doctor terminal 70 may execute part or all of the processing in FIG. 10. For example, the processing in FIG. 10 need only be implemented by one or a plurality of devices in the information processing system 100.

Note that, in the embodiment described above, the case has been described where the sensing device 50 is connected to the physical therapist terminal 60, but the present invention is not limited to this, and the sensing device 50 may be connected to the doctor terminal 70. In this case, the doctor terminal 70 need only perform processing similar to the processing performed by the physical therapist terminal 60 of the embodiment described above.

Note that, in the embodiment described above, the case has been described where the output unit 34 outputs the feature amount to the doctor terminal 70, but the present invention is not limited to this, and the output unit 34 may output the feature amount to other terminals. For example, the output unit 34 may output the feature amount to the physical therapist terminal 60 or a terminal of a pharmaceutical company conducting a clinical trial.

Note that the processing functions described above may be implemented by a computer. In that case, a program is provided in which processing content of a function that a processing device should have is described. The program is executed on the computer, whereby the processing functions described above are implemented on the computer. The program in which the processing content is described may be recorded in a computer-readable storage medium (note that a carrier wave is excluded).

In a case of distributing the program, for example, the program is sold in a form of a portable storage medium such as a digital versatile disc (DVD) or a compact disc read only memory (CD-ROM) in which the program is recorded. Furthermore, it is also possible to store the program in a storage device of a server computer and transfer the program from the server computer to another computer via a network.

The computer that executes the program stores, for example, the program recorded in the portable storage medium or the program transferred from the server computer in a storage device of the computer. Then, the computer reads the program from the storage device of the computer and executes processing according to the program. Note that the computer may also read the program directly from the portable storage medium and execute processing according to the program. Furthermore, the computer may also sequentially execute processing according to the received program each time the program is transferred from the server computer.

The embodiment described above is an example of a preferred embodiment of the present invention. However, the present invention is not limited to this, and various modifications may be made without departing from the scope of the present invention, which is defined by the appended claims.

[Reference Signs List]

| | |
|---|---|
| 20 | Sensing result acquisition unit (Acquisition unit) |
| 50 | Sensing device (Sensor) |
| 24 | Initial contact detection unit (Detection unit) |
| 26 | Search width setting unit (Specification unit) |
| 28 | Peak value search unit (Part of calculation unit) |
| 30 | Toe gait determination unit (Part of calculation unit) |
| 32 | Feature amount calculation unit (Part of calculation unit) |
| 100 | Information processing system |

**Claims**

1. An information processing program comprising instructions which, when executed by a computer, cause said computer (60) to execute processing of:

   acquiring information regarding an angular velocity around a first axis that extends in a right and left direction of a person and an acceleration in a front and back direction of the person during walking, which are detected by a sensor attached to an ankle of the person;
   detecting a timing when a foot that is off a ground touches the ground based on change in the angular velocity over time around the first axis;
   specifying, as a time of interest, a predetermined time before and after the detected timing; and
   calculating an index based on change in the acceleration in the front and back direction within the time of interest, and
   determining
   whether or not the person is performing a toe gait using said index.

2. The information processing program according to claim 1, wherein the processing of calculating includes:

   specifying a change pattern of the acceleration in the front and back direction in each of a plurality of the times of interest, and
   calculating a percentage that the specified change pattern is a predetermined change pattern as the index.

3. The information processing program according to claim 2, wherein the predetermined change pattern is a pattern in which, before a local maximum value that exceeds a predetermined reference range, a local minimum value that is lower than the reference range appears, and after the local maximum value, a local minimum value that is lower than the reference range does not appear.

4. The information processing program according to claim 1, wherein the processing of calculating includes:

   calculating, in a case where, within the time of interest, a change pattern of the acceleration in the front and back direction is a pattern in which, before a local maximum value that exceeds a predetermined reference range, a first local minimum value that is lower than the reference range appears, and after the local maximum value, a second local minimum value that is lower than the reference range or within the reference range appears, a ratio of a difference between the local maximum value and the first local minimum value and a difference between the local maximum value and the second local minimum value as the index.

5. An information processing method implemented by a computer (60), the information processing method comprising processing of:

   acquiring information regarding an angular velocity around a first axis that extends in a right and left direction of a person and an acceleration in a front and back direction of the person during walking, which are detected by a sensor attached to an ankle of the person;
   detecting a timing when a foot that is off a ground touches the ground based on change in the angular velocity over time around the first axis;
   specifying, as a time of interest, a predetermined time before and after the detected timing; and
   calculating an index based on change in the acceleration in the front and back direction within the time of interest, and
   determining whether or not the person is performing a toe gait using said index.

6. An information processing system (100) comprising:

   an acquisition unit (20) configured to acquire information regarding an angular velocity around a first axis that extends in a right and left direction of a person and an acceleration in a front and back direction of the person during walking, which are detected by a sensor attached to an ankle of the person;
   a detection unit (24) configured to detect a timing when a foot that is off a ground touches the ground based on change in the angular velocity over time around the first axis;
   a specification unit (26) configured to specify, as a time of interest, a predetermined time before and after the detected timing; and

a calculation unit (28, 30, 32) configured to calculate an index based on change in the acceleration in the front and back direction within the time of interest, and to determine whether or not the person is performing a toe gait using said index.

7. The information processing system (100) according to claim 6, wherein the calculation unit (28, 30, 32) is configured to specify a change pattern of the acceleration in the front and back direction in each of a plurality of the times of interest, and to calculate a percentage that the specified change pattern is a predetermined change pattern as the index.

8. The information processing system (100) according to claim 7, wherein the predetermined change pattern is a pattern in which, before a local maximum value that exceeds a predetermined reference range, a local minimum value that is lower than the reference range appears, and after the local maximum value, a local minimum value that is lower than the reference range does not appear.

9. The information processing system (100) according to claim 6, wherein, in a case where, within the time of interest, a change pattern of the acceleration in the front and back direction is a pattern in which, before a local maximum value that exceeds a predetermined reference range, a first local minimum value that is lower than the reference range appears, and after the local maximum value, a second local minimum value that is lower than the reference range or within the reference range appears, the calculation unit (28, 30, 32) calculates a ratio of a difference between the local maximum value and the first local minimum value and a difference between the local maximum value and the second local minimum value as the index.

**Patentansprüche**

1. Informationsverarbeitungsprogramm, umfassend Anweisungen, die, wenn sie von einem Computer ausgeführt werden, bewirken, dass der Computer (60) Verarbeitung ausführt von:

   Erfassen von Informationen in Bezug auf eine Winkelgeschwindigkeit um eine erste Achse herum, die sich in eine rechte und linke Richtung einer Person erstreckt, und eine Beschleunigung in eine Vorwärts- und Rückwärtsrichtung der Person während Gehens, die von einem Sensor erkannt werden, der an einem Knöchel der Person befestigt ist;
   Erkennen eines Zeitpunkts, wenn ein Fuß, der von einem Boden abgehoben ist, den Boden berührt, basierend auf Veränderung der Winkelgeschwindigkeit im Laufe der Zeit um die erste Achse herum;
   Spezifizieren, als eine interessierende Zeit, einer vorbestimmten Zeit vor und nach dem erkannten Zeitpunkt; und
   Berechnen eines Indexes basierend auf Veränderung der Beschleunigung in die Vorwärts- und Rückwärtsrichtung innerhalb der interessierenden Zeit,
   und Bestimmen unter Verwendung des Indexes, ob die Person einen Fußspitzengang durchführt oder nicht.

2. Informationsverarbeitungsprogramm nach Anspruch 1, wobei die Berechnungsverarbeitung Folgendes einschließt:

   Spezifizieren eines Veränderungsmusters der Beschleunigung in die Vorwärts- und Rückwärtsrichtung bei jeder einer Vielzahl von den interessierenden Zeiten, und
   Berechnen eines Prozentsatzes, bei dem das spezifizierte Veränderungsmuster ein vorbestimmtes Veränderungsmuster ist, als den Index.

3. Informationsverarbeitungsprogramm nach Anspruch 2, wobei das vorbestimmte Veränderungsmuster ein Muster ist, bei dem vor einem lokalen Maximalwert, der einen vorbestimmten Referenzbereich übersteigt, ein lokaler Minimalwert auftritt, der niedriger ist als der Referenzbereich, und nach dem lokalen Maximalwert ein lokaler Minimalwert, der niedriger ist als der Referenzbereich, nicht auftritt.

4. Informationsverarbeitungsprogramm nach Anspruch 1, wobei die Berechnungsverarbeitung Folgendes einschließt:
   Berechnen in einem Fall, in dem innerhalb der interessierenden Zeit ein Veränderungsmuster der Beschleunigung in die Vorwärts- und Rückwärtsrichtung ein Muster ist, bei dem vor einem lokalen Maximalwert, der einen vorbestimmten Referenzbereich übersteigt, ein erster lokaler Minimalwert auftritt, der niedriger ist als der Referenzbereich, und nach dem lokalen Maximalwert ein zweiter lokaler Minimalwert auftritt, der niedriger ist als der Referenzbereich oder innerhalb des Referenzbereichs ist, eines Verhältnisses einer Differenz zwischen dem lokalen Maximalwert und dem ersten lokalen Minimalwert und einer Differenz zwischen dem lokalen Maximalwert und dem zweiten lokalen Minimalwert als den Index.

5. Informationsverarbeitungsverfahren, implementiert durch einen Computer (60), wobei das Informationsverarbeitungsverfahren Verarbeiten umfasst von:

Erfassen von Informationen in Bezug auf eine Winkelgeschwindigkeit um eine erste Achse herum, die sich in eine rechte und linke Richtung einer Person erstreckt, und eine Beschleunigung in eine Vorwärts- und Rückwärtsrichtung der Person während Gehens, die von einem Sensor erkannt werden, der an einem Knöchel der Person befestigt ist;

Erkennen eines Zeitpunkts, wenn ein Fuß, der von einem Boden abgehoben ist, den Boden berührt, basierend auf Veränderung der Winkelgeschwindigkeit im Laufe der Zeit um die erste Achse herum;

Spezifizieren, als eine interessierende Zeit, einer vorbestimmten Zeit vor und nach dem erkannten Zeitpunkt; und

Berechnen eines Indexes basierend auf Veränderung der Beschleunigung in die Vorwärts- und Rückwärtsrichtung innerhalb der interessierenden Zeit,

und Bestimmen unter Verwendung des Indexes, ob die Person einen Fußspitzengang durchführt oder nicht.

6.   Informationsverarbeitungssystem (100), umfassend:

eine Erfassungseinheit (20), die konfiguriert ist, um Informationen in Bezug auf eine Winkelgeschwindigkeit um eine erste Achse herum, die sich in eine rechte und linke Richtung einer Person erstreckt, und eine Beschleunigung in eine Vorwärts- und Rückwärtsrichtung der Person während Gehens, die von einem Sensor erkannt werden, der an einem Knöchel der Person befestigt ist, zu erfassen;

eine Erkennungseinheit (24), die konfiguriert ist, um einen Zeitpunkt zu erkennen, wenn ein Fuß, der von einem Boden abgehoben ist, den Boden berührt, basierend auf Veränderung der Winkelgeschwindigkeit im Laufe der Zeit um die erste Achse herum;

eine Spezifizierungseinheit (26), die konfiguriert ist, um als eine interessierende Zeit eine vorbestimmte Zeit vor und nach dem erkannten Zeitpunkt zu spezifizieren; und

eine Berechnungseinheit (28, 30, 32), die konfiguriert ist, um einen Index basierend auf Veränderung der Beschleunigung in die Vorwärts- und Rückwärtsrichtung innerhalb der interessierenden Zeit zu berechnen, und unter Verwendung des Indexes zu bestimmen, ob die Person einen Fußspitzengang durchführt oder nicht.

7.   Informationsverarbeitungssystem (100) nach Anspruch 6, wobei die Berechnungseinheit (28, 30, 32) konfiguriert ist, um ein Veränderungsmuster der Beschleunigung in die Vorwärts- und Rückwärtsrichtung in jeder einer Vielzahl von den interessierenden Zeiten zu spezifizieren, und einen Prozentsatz, bei dem das spezifizierte Veränderungsmuster ein vorbestimmtes Veränderungsmuster ist, als den Index zu berechnen.

8.   Informationsverarbeitungssystem (100) nach Anspruch 7, wobei das vorbestimmte Veränderungsmuster ein Muster ist, bei dem vor einem lokalen Maximalwert, der einen vorbestimmten Referenzbereich übersteigt, ein lokaler Minimalwert auftritt, der niedriger ist als der Referenzbereich, und nach dem lokalen Maximalwert ein lokaler Minimalwert, der niedriger ist als der Referenzbereich, nicht auftritt.

9.   Informationsverarbeitungssystem (100) nach Anspruch 6, wobei, in einem Fall, in dem innerhalb der interessierenden Zeit ein Veränderungsmuster der Beschleunigung in die Vorwärts- und Rückwärtsrichtung ein Muster ist, bei dem vor einem lokalen Maximalwert, der einen vorbestimmten Referenzbereich übersteigt, ein erster lokaler Minimalwert auftritt, der niedriger ist als der Referenzbereich, und nach dem lokalen Maximalwert ein zweiter lokaler Minimalwert auftritt, der niedriger ist als der Referenzbereich oder innerhalb des Referenzbereichs ist, die Berechnungseinheit (28, 30, 32) ein Verhältnis einer Differenz zwischen dem lokalen Maximalwert und dem ersten lokalen Minimalwert und einer Differenz zwischen dem lokalen Maximalwert und dem zweiten lokalen Minimalwert als den Index berechnet.

**Revendications**

1.   Programme de traitement d'informations comprenant des instructions qui, lorsqu'elles sont exécutées par un ordinateur, amènent ledit ordinateur (60) à exécuter un traitement de :

acquisition d'informations concernant une vitesse angulaire autour d'un premier axe qui s'étend dans une direction droite et gauche d'une personne et une accélération dans une direction avant et arrière de la personne pendant qu'elle marche, qui sont détectées par un capteur attaché à une cheville de la personne ;

détection d'un moment où un pied qui est décollé d'un sol touche le sol sur la base d'un changement de la vitesse angulaire au fil du temps autour du premier axe ;

spécification, en tant que temps d'intérêt, d'un temps prédéterminé avant et après le moment détecté ; et

calcul d'un indice sur la base d'un changement de l'accélération dans la direction avant et arrière dans le temps d'intérêt,

et action de déterminer si la personne effectue ou non une marche sur la pointe des pieds à l'aide dudit indice.

2. Programme de traitement d'informations selon la revendication 1, dans lequel le traitement de calcul inclut :

la spécification d'un profil de changement de l'accélération dans la direction avant et arrière dans chacun d'une pluralité de temps d'intérêt, et

le calcul d'un pourcentage selon lequel le profil de changement spécifié est un profil de changement prédéterminé en tant qu'indice.

3. Programme de traitement d'informations selon la revendication 2, dans lequel le profil de changement prédéterminé est un profil dans lequel, avant une valeur maximale locale qui dépasse une plage de référence prédéterminée, une valeur minimale locale qui est inférieure à la plage de référence apparaît, et après la valeur maximale locale, une valeur minimale locale qui est inférieure à la plage de référence n'apparaît pas.

4. Programme de traitement d'informations selon la revendication 1, dans lequel le traitement de calcul inclut :
le calcul, dans un cas où, dans le temps d'intérêt, un profil de changement de l'accélération dans la direction avant et arrière est un profil dans lequel, avant une valeur maximale locale qui dépasse une plage de référence prédéterminée, une première valeur minimale locale qui est inférieure à la plage de référence apparaît, et après la valeur maximale locale, une seconde valeur minimale locale qui est inférieure à la plage de référence ou dans la plage de référence apparaît, un rapport d'une différence entre la valeur maximale locale et la première valeur minimale locale et d'une différence entre la valeur maximale locale et la seconde valeur minimale locale en tant qu'indice.

5. Procédé de traitement d'informations mis en oeuvre par un ordinateur (60), le procédé de traitement d'informations comprenant un traitement de :

acquisition d'informations concernant une vitesse angulaire autour d'un premier axe qui s'étend dans une direction droite et gauche d'une personne et une accélération dans une direction avant et arrière de la personne pendant qu'elle marche, qui sont détectées par un capteur attaché à une cheville de la personne ;
détection d'un moment où un pied qui est décollé d'un sol touche le sol sur la base d'un changement de la vitesse angulaire au fil du temps autour du premier axe ;
spécification, en tant que temps d'intérêt, d'un temps prédéterminé avant et après le moment détecté ; et
calcul d'un indice sur la base d'un changement de l'accélération dans la direction avant et arrière dans le temps d'intérêt, et
action de déterminer si la personne effectue ou non une marche sur la pointe des pieds à l'aide dudit indice.

6. Système de traitement d'informations (100) comprenant :

une unité d'acquisition (20) configurée pour acquérir des informations concernant une vitesse angulaire autour d'un premier axe qui s'étend dans une direction droite et gauche d'une personne et une accélération dans une direction avant et arrière de la personne pendant qu'elle marche, qui sont détectées par un capteur attaché une cheville de la personne ;
une unité de détection (24) configurée pour détecter un moment où un pied qui est décollé d'un sol touche le sol sur la base d'un changement de la vitesse angulaire au fil du temps autour du premier axe ;
une unité de spécification (26) configurée pour spécifier, en tant que temps d'intérêt, un temps prédéterminé avant et après le moment détecté ; et
une unité de calcul (28, 30, 32) configurée pour calculer un indice sur la base d'un changement de l'accélération dans la direction avant et arrière dans le temps d'intérêt, et pour déterminer si la personne effectue ou non une marche sur la pointe des pieds à l'aide dudit indice.

7. Système de traitement d'informations (100) selon la revendication 6, dans lequel l'unité de calcul (28, 30, 32) est configurée pour spécifier un profil de changement de l'accélération dans la direction avant et arrière dans chacun d'une pluralité des temps d'intérêt, et pour calculer un pourcentage selon lequel le profil de changement spécifié est un profil de changement prédéterminé en tant qu'indice.

8. Système de traitement d'informations (100) selon la revendication 7, dans lequel le profil de changement prédéterminé est un profil dans lequel, avant une valeur maximale locale qui dépasse une plage de référence prédéterminée, une valeur minimale locale qui est inférieure à la plage de référence apparaît, et après la valeur maximale locale, une valeur minimale locale qui est inférieure à la plage de référence n'apparaît pas.

9. Système de traitement d'informations (100) selon la revendication 6, dans lequel, dans un cas où, dans le temps d'intérêt, un profil de changement de l'accélération dans la direction avant et arrière est un profil dans lequel, avant une valeur maximale locale qui dépasse une plage de référence prédéterminée, une première valeur minimale locale qui est inférieure à la plage de référence apparaît, et après la valeur maximale locale, une seconde valeur minimale locale qui est inférieure à la plage de référence ou dans la plage de référence apparaît, l'unité de calcul (28, 30, 32) calcule un rapport d'une différence entre la valeur maximale locale et la première valeur minimale locale et d'une différence entre la valeur maximale locale et la seconde valeur minimale locale en tant qu'indice.

# FIG. 1

# FIG. 2A

60,70

- CPU 190
- ROM 192
- RAM 194
- HDD 196
- NETWORK INTERFACE 197
- PORTABLE STORAGE MEDIUM DRIVE 199
- PORTABLE STORAGE MEDIUM 191
- DISPLAY UNIT 193
- INPUT UNIT 195
- 198

# FIG. 2B

# FIG. 3

| | 0% INITIAL CONTACT IR | 10% OPPOSITE TOE OFF | 30% HEEL RAISE | | 50% OPPOSITE INITIAL CONTACT | 60% TOE OFF | 73% FEET ADJACENT | 87% TIBIA VERTICAL | 100% NEXT INITIAL CONTACT |
|---|---|---|---|---|---|---|---|---|---|

| PERIODS | LR LOADING RESPONSE | Mst MID STANCE | Tst TERMINAL STANCE | Psw PRE- SWING | Isw INITIAL SWING | Msw MID SWING | Tsw TERMINAL SWING |
|---|---|---|---|---|---|---|---|
| PHASES | STANCE PHASE | | | | SWING PHASE | | |
| TASKS | WEIGHT ACCEPTANCE | SINGLE-LIMB SUPPORT | | | LIMB ADVANCEMENT | | |
| CYCLES | RIGHT LEG CYCLE | | | | | | |

EP 3 915 473 B1

# FIG. 4

SENSING RESULT ACQUISITION UNIT — 20

INITIAL CONTACT DETECTION UNIT — 24

SEARCH WIDTH SETTING UNIT — 26

PEAK VALUE SEARCH UNIT — 28

TOE GAIT DETERMINATION UNIT — 30

FEATURE AMOUNT CALCULATION UNIT — 32

OUTPUT UNIT — 34

# FIG. 5

FIG. 6A

ANGULAR VELOCITY (AROUND X-AXIS)

FIG. 6B

ACCELERATION (Y-AXIS)

EP 3 915 473 B1

## FIG. 7A

ANGULAR VELOCITY
(AROUND X-AXIS)

ANGULAR VELOCITY [deg/sec]

400
200
0
-200
-400

INITIAL CONTACT    ELAPSED TIME

## FIG. 7B

ACCELERATION
(Y-AXIS)

ANGULAR VELOCITY [deg/sec]

40
20
0
-20
-40

LOCAL MAXIMUM VALUE

SEARCH WIDTH

LOCAL MINIMUM VALUE    LOCAL MINIMUM VALUE    ELAPSED TIME

## FIG. 7C

HEEL IS FIXED BY HEEL LANDING

SHIN MOVES FORWARD WITH HEEL FIXED

TOE IS FIXED BY TOE LANDING

## FIG. 8A

ANGULAR VELOCITY
(AROUND X-AXIS)

ANGULAR VELOCITY [deg/sec]

400

200

0

-200

-400

INITIAL CONTACT    ELAPSED TIME

## FIG. 8B

ACCELERATION
(Y-AXIS)

ANGULAR VELOCITY [deg/sec]

40

20

0

-20

-40

SEARCH
WIDTH

LOCAL MAXIMUM
VALUE

LOCAL
MINIMUM
VALUE

ELAPSED TIME

## FIG. 8C

TOE IS FIXED BY
TOE LANDING

SHIN MOVES FORWARD
WITH TOE FIXED

# FIG. 9

<PREPROCESSING>

```
        ┌─────────┐
        │  START  │
        └─────────┘
             │
             ▼
      ╱───────────────╲  ┌S10
NO ◄─┤  IS MEASUREMENT  ├
      ╲  TO BE STARTED? ╱
       ╲───────────────╱
             │ YES
             ▼
      ╱───────────────╲  ┌S12
NO ◄─┤ HAS PREDETERMINED├
      ╲  TIME ELAPSED?  ╱
       ╲───────────────╱
             │ YES          ┌S14
    ┌────────────────────────────┐
    │ DETECT ANGULAR VELOCITY     │
    │ AROUND X-AXIS AND           │
    │ ACCELERATION IN Y-AXIS      │
    │ DIRECTION, AND STORE ANGULAR│
    │ VELOCITY AND ACCELERATION   │
    │ TOGETHER WITH TIME          │
    │ INFORMATION                 │
    └────────────────────────────┘
             │
             ▼
      ╱───────────────────────╲  ┌S16
     ┤ IS MEASUREMENT TO BE ENDED? ├─► NO
      ╲───────────────────────╱
             │ YES
             ▼
        ┌─────────┐
        │   END   │
        └─────────┘
```

# FIG. 10

<DATA ANALYSIS PROCESSING>

START

ACQUIRE DATA OF ANGULAR VELOCITY AROUND X-AXIS AND DATA OF ACCELERATION IN Y-AXIS DIRECTION OF TARGET PATIENT — S30

DETECT INITIAL CONTACT FROM CHANGE IN ANGULAR VELOCITY AROUND X-AXIS — S32

SET 250 ms BEFORE AND AFTER INITIAL CONTACT AS SEARCH WIDTH — S34

SEARCH FOR PEAK VALUES OF ACCELERATION IN Y-AXIS DIRECTION WITHIN SEARCH WIDTH — S36

S38

NO — HAS SEARCH WITHIN ALL SEARCH WIDTHS BEEN COMPLETED?

YES

OBTAIN THE NUMBER OF SEARCH WIDTHS IN WHICH LOCAL MINIMUM VALUE APPEARS ONLY BEFORE LOCAL MAXIMUM VALUE WITHIN EACH SEARCH WIDTH — S40

USE, AS FEATURE AMOUNT OF TARGET PATIENT, VALUE OBTAINED BY DIVIDING THE OBTAINED NUMBER OF SEARCH WIDTHS BY TOTAL NUMBER OF STEPS — S42

OUTPUT FEATURE AMOUNT OF TARGET PATIENT — S44

END

# FIG. 11A

ANGULAR VELOCITY (AROUND X-AXIS)

# FIG. 11B

ACCELERATION (Y-AXIS)

SEARCH WIDTH

TOE (ONE STEP)
NORMAL (TWO STEPS)
TOE (THREE STEPS)
TOE (FOUR STEPS)
TOE (FIVE STEPS)
NORMAL (SIX STEPS)
TOE (SEVEN STEPS)
TOE (EIGHT STEPS)

# FIG. 12A

**SUBJECT A**

# FIG. 12B

**SUBJECT B**

## FIG. 13A

## FIG. 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013108306 A
- EP 3257437 A1

- US 2018263532 A1

### Non-patent literature cited in the description

- Inertial Sensor-Based Stride Parameter Calculation From Gait Sequences in Geriatric Patients. **RAMPP ALEXANDER et al.** IEEE TRANSACTIONS ON BIOMEDICAL ENGINEERING. IEEE, 01 April 2015, vol. 62

- **PENDHARKAR GITA et al.** Automated method to distinguish toe walking strides from normal strides in the gait of idiopathic toe walking children from heel accelerometry data. *GAIT & POSTURE,* vol. 35 (3), 478-482